# EUROPEAN PATENT APPLICATION

(11) **EP 1 949 801 A1**
(43) Date de publication de la demande: **30.07.2008**
(21) Numéro de dépôt: 06380302.7
(22) Date de dépôt: 17.11.2006
(51) Int. Cl.: A23L 1/308, A61K 31/695, A61K 33/00, A23L 2/02

(54) **Boisson nutritive et régénératrice**

(71) Demandeur: Fernandez Jimenez, Maria Ascension, 28660 Boadilla del Monte, Madrid (ES)
(72) Inventeur: Fernandez Jimenez, Maria Ascension, 28660 Boadilla del Monte, Madrid (ES)
(74) Mandataire: Manzano Cantos, Gregorio

(57) **Abrégé**

UNE BOISSON NUTRITIVE ET RÉGÉNÉRATRICE, qui consiste en une composition de jus concentrés de fruits naturels à base de jus concentré d'orange; jus concentré de mandarine; jus concentré de raisin, dissous dans de l'eau purifiée à laquelle on ajoute un oligo-élément et une vitamine.

## Description

### OBJET DE L'INVENTION

L'invention consiste en une boisson nutritive et régénératrice de l'appareil locomoteur composée à base de jus concentré de fruits, dans laquelle intervient un fruit et un oligo-élément ou ingrédient essentiel pour stimuler le cartilage articulaire du corps humain dans sa fonction biologique vitale pour assurer l'apport quotidien nécessaire de cet oligo-élément.

Il s'agit d'une boisson 100% naturelle, qui inclut du jus concentré de fruits tels que: orage, mandarine, raison; eau purifiée à laquelle on ajoute de la vitamine C (acide ascorbique) et un supplément essentiel comme oligo-élément tel que du silicium. Sans addition de sucre, non carbonatée et sans colorants artificiels.

Toutes les personnes, comme l'on sait, ont dépôts de silicium organique, lesquels avec l'âge se réduisent, si l'on ne l'inclut pas à travers de l'alimentation son manque peut nous faire subir diverses maladies. En l'incluant dans une boisson à base de jus de fruits concentrés dans les conditions proposées par l'invention, avec la quantité d'une canette, bouteille, récipient de 250 ml. Nous serons sûrs de maintenir les niveaux stables de silicium pour le cartilage articulaire.

### ANTÉCÉDENTS DE L'INVENTION

II y a plusieurs boissons stimulantes ou tonifiantes d'effet musculaire qui sont utilisées surtout pour compenser la détérioration musculaire pour lesdites raisons de pertes dues à l'âge, de l'effort sportif ou d'une toute autre cause, incorporant une substance pour assurer une bonne fonction de la boisson et un bon résultat pour stimuler différents organes articulaires du corps.

Le silicium buvable à l'état liquide est connu, aux États Unis, mais la molécule a été synthétisée en France et on ne connaît pas son utilisation dans une combinaison également buvable de la nature proposée par l'invention, dans laquelle le facteur stimulant et tonifiant desdites substances est combinée avec ledit oligo-élément et dans les conditions proposées par l'invention.

### DESCRIPTION DE L'INVENTION

L'objet de l'invention est, comme il a été dit, une boisson à base de jus concentré à de fruits, composée d'orange, mandarine, raisin, eau purifiée, à laquelle on ajoute de la vitamine C et un oligo-élément à base de silicium manquant d'addition de sucre, non-carbonatée, sans colorants artificiels. Il s'agit par conséquent d'une boisson à l'état naturelle dans laquelle ils interviennent à 100% dans une composition:
25 % de jus d'orange
10 % de jus de mandarine
5 % de jus de raisin
40-41 % d'eau purifiée
0,02% de Vitamine C
0,12- 0,24 % de Silicium (Si) G5

Les composants sont dissous dans l'eau purifiée, en les agitant et en les laissant reposer jusqu'à la mise en bouteille finale. On les fournit et vend dans le marché, protégés à une température appropriée de demi-conserve.

Un autre détail de l'invention est le fait que, pour une composition d'une portion de boisson de 250 ml, le pourcentage de capacité de chacun des ingrédients serait de:
62,50 ml de jus d'orange
25 ml de jus de mandarine
12,5 ml de jus de raisin
100- 102,50 ml d'eau purifiée
0,05 ml de vitamine C
0,30-0,60 ml de silicium liquide G5

Comme il a été dit, le silicium buvable est connu, mais il n'a pas un goût organoleptique, il est désagréable et il faudrait des recommandations ou des instructions pour le faire en combinaison avec d'autres boissons, selon la boisson de l'invention le goût est amélioré et il y a un apport d'un index élevé d'hydratation, des fruits riches en vitamines et minéraux, de la vitamine C fondamental pour prévenir des refroidissements.

Comme l'on sait la vitamine C ou acide ascorbique est une vitamine soluble dans l'eau, il s'accumule à peine dans l'organisme, doit être ingéré quotidiennement à travers la diète selon les besoins individuels.

La boisson objet de l'invention a les fonctions bio-organiques suivantes:
- Augmentation de l'absorption organique du fer présent dans les aliments. Elle intervient dans la formation du collagène, constituant principal du cartilage de l'os. Participation à l'intégrité de gencives, os, dents, vaisseaux sanguins, à la synthèse d'hormones stéroïdiennes et au métabolisme des graisses (lipides).
- Influence sur l'activité des leucocytes et macrophages, cellules composant le système de défense de l'organisme. Participation active aux procédés de désintoxication se produisant dans le foie.
- Aide à la cicatrisation de blessures, a des effets anti-oxydants contre l'action nuisible des radicaux libres, liés au développement de la maladie tumorale.
- Inhibition de la formation de nitrosamines (substances potentiellement cancérigènes) dans l'estomac à partir des nitrates, substances qui se trouvent présentes dans certaines boissons et certains aliments.

Comme on peut en déduire du contenu de l'invention, le silicium organique aide le métabolisme cellulaire, régule le potentiel électrique en rééquilibrant les ions positifs et négatifs et facilite la transmission de la communication intercellulaire en lui apportant l'énergie nécessaire pour sa fonction. C'est un fortifiant des défenses de l'organisme. C'est un élément essentiel dans la formation des os et des cartilages, dans la santé du cerveau pour éviter la dégénération propre de l'âge, si on ajoute une diète basse en calcium, il influe sur la santé cardiovasculaire, il prévient le développement de la plaque athérome, il assure l'imperméabilité des parois artérielles aux dépôts de lipides, outre l'intégrité des fibres élastiques.

## Revendications

1. UNE BOISSON NUTRITIVE ET RÉGÉNÉRATRICE, constituée par un concentré de jus de fruits naturels **caractérisée en ce qu'**elle comprend l'apport essentiel d'un oligo-élément à base de silicium liquide en lui ajoutant de la vitamine C qui sera dissoute dans un concentré de fruits intégré par:
du jus concentré d'orange;
du jus concentré de mandarine;
du jus concentré de raisin;
dissous dans de l'eau purifiée.

2. UNE BOISSON NUTRITIVE ET RÉGÉNÉRATRICE, selon la revendication antérieure, l'apport de silicium étant **caractérisé en ce que** c'est du silicium liquide de 5^{ème} génération avec une participation de: 0,12 - 0,24 %.

3. UNE BOISSON NUTRITIVE ET RÉGÉNÉRATRICE, selon la revendication 1, l'apport de vitamine C étant **caractérisé en ce que** c'est une vitamine à l'état liquide avec une participation de 0,02 %.

4. UNE BOISSON NUTRITIVE ET RÉGÉNÉRATRICE, selon les revendications 1 à 3, la concentration à 100% de la composition étant **caractérisée en ce qu'**elle a la participation suivante:
25 % de jus d'orange
10 % de jus de mandarine
5 % de jus de raisin
40-41 % d'eau purifiée
0, 02% de Vitamine C
0,12- 0,24 % de Silicium (Si) G5

5. UNE BOISSON NUTRITIVE ET RÉGÉNÉRATRICE, selon les revendications 1 à 3, la concentration quantitative de la composition étant **caractérisée en ce qu'**elle a la proportion suivante pour une dose de 250 ml:
62,50 ml de jus d'orange
25 ml jus de mandarine
12,5 ml jus de raisin
100- 102,50 ml d'eau purifiée
0,05 ml vitamine C
0,30-0,60 ml silicium liquide G5

## Revendications modifiées

### Revendications modifiées conformément à la règle 137(2) CBE.

**1.** UNE BOISSON NUTRITIVE ET RÉGÉNÉRATRICE, constituée par un avec une participation de 0,02 % de jus de fruits naturels concentrés **caractérisée en ce qu'**elle comprend l'apport essentiel d'un oligo-élément à base de silicium liquide en lui ajoutant de la vitamine C qui sera dissoute dans le concentré de fruits intégré par:
du jus concentré d'orange;
du jus concentré de mandarine;
du jus concentré de raisin;
dissout dans de l'eau purifiée.

**2.** UNE BOISSON NUTRITIVE ET RÉGÉNÉRATRICE, selon la revendication antérieure, l'apport de silicium étant **caractérisé en ce que** c'est avec une participation proportionnelle de: 0,12 - 0,24 % du silicium liquide de 5^{ème} génération.

**3.** UNE BOISSON NUTRITIVE ET RÉGÉNÉRATRICE, selon la revendication 1, l'apport de vitamine C étant **caractérisé en ce que** c'est avec une participation proportionnelle de 0,02 % à l'état liquide.

**4.** UNE BOISSON NUTRITIVE ET RÉGÉNÉRATRICE, selon les revendications 1 à 3, la concentration à 100% de la boisson étant **caractérisée en ce qu'**elle a la participation proportionnelle suivante:
25 % de jus d'orange
10 % de jus de mandarine
5 % de jus de raisin
40-41 % d'eau purifiée
0, 02% de Vitamine C
0,12- 0,24 % de Silicium (Si) G5

**5.** UNE BOISSON NUTRITIVE ET RÉGÉNÉRATRICE, selon les revendications 1 à 3, la concentration quantitative de la composition étant **caractérisée en ce qu'**elle a la proportion pour une dose de 250 ml est:
62,50 ml de jus d'orange
25 ml jus de mandarine
12,5 ml jus de raisin
100- 102,50 ml d'eau purifiée
0,05 ml vitamine C
0,30-0,60 ml silicium liquide G5
